# EUROPEAN PATENT APPLICATION

(11) **EP 4 470 447 A1**
(43) Date of publication of application: **04.12.2024**
(21) Application number: 23746694.1
(22) Date of filing: 13.01.2023
(51) Int. Cl.: A61B 1/00, G02B 7/28, G02B 7/36, G03B 13/36, G03B 15/00, H04N 23/67

(54) **MEDICAL IMAGING SYSTEM, CONTROL METHOD, AND PROGRAM**

(30) Priority: 28.01.2022 JP 2022011903
(71) Applicant: Sony Group Corporation, Tokyo 108-0075 (JP)
(72) Inventor: ONIKUBO Masatoshi, Tokyo 108-0075 (JP)
(74) Representative: D Young & Co LLP
(86) International application number: PCT/JP2023/000721
(87) International publication number: WO 2023/145480

(57) **Abstract**

The present disclosure relates to a medical imaging system, a control method, and a program capable of coping with a case where there is a plurality of regions desired to be focused.

Provided is a medical imaging system including a medical imaging device including an assignable button, and configured to image an operative field during surgery to generate a surgical video signal, and a control device configured to control the medical imaging device, in which the control device includes one or more processors and one or more storage devices storing a program, and the processor executes the program to calculate a plurality of AF evaluation values on the basis of the surgical video signal output from the medical imaging device, calculate focus lens moving positions corresponding to respective AF evaluation values on the basis of the plurality of calculated AF evaluation values, and set at least two of the calculated focus lens moving positions for the assignable button. The present disclosure is applicable to, for example, an endoscopic surgery system and the like.

## Description

### TECHNICAL FIELD

The present disclosure relates to a medical imaging system, a control method, and a program, and more particularly, to a medical imaging system, a control method, and a program capable of coping with a case where there is a plurality of regions desired to be focused.

### BACKGROUND ART

In general, in a medical observation device such as a surgical endoscope or a surgical microscope, a depth of field thereof is shallow, while an operative field has a depth, so that a portion desired to be visually recognized may not be focused. On the other hand, a medical observation device having an auto focus (AF) function of automatically focusing a focus has been proposed.

For example, Patent Document 1 discloses that a highly accurate AF function is realized by devising a wobbling operation in an observation mode in which light having different wavelengths is alternately radiated.

### CITATION LIST

### PATENT DOCUMENT

Patent Document 1: Japanese Patent Application Laid-Open No. 2021-157067

### SUMMARY OF THE INVENTION

### PROBLEMS TO BE SOLVED BY THE INVENTION

Meanwhile, there may be a plurality of attention points in surgery. When there is a plurality of attention points, the surgical staff member performs surgery while alternately looking at a plurality of attention points.

As described above, in a case where there is a plurality of attention points, it is assumed that there is a plurality of regions desired to be focused, but it is difficult for the current AF function on the assumption that there is one region desired to be focused to cope with the case. Therefore, it is required to provide an AF function that can cope with a case where there is a plurality of regions desired to be focused.

The present disclosure has been made in view of such a situation, and can cope with a case where there is a plurality of regions desired to be focused.

### SOLUTIONS TO PROBLEMS

A medical imaging system according to an aspect of the present disclosure includes a medical imaging device including an assignable button to which execution of a selected function is assignable and configured to image an operative field during surgery to generate a surgical video signal, and a control device configured to control the medical imaging device, in which the control device includes one or more processors and one or more storage devices storing a program, and the processor executes the program to calculate a plurality of AF evaluation values on the basis of the surgical video signal output from the medical imaging device, calculate a focus lens moving position corresponding to respective AF evaluation values on the basis of the plurality of calculated AF evaluation values, and set at least two of the calculated focus lens moving positions for the assignable button.

A control method according to an aspect of the present disclosure is a control method including a control device configured to control a medical imaging device including an assignable button to which execution of a selected function is assignable and configured to image an operative field during surgery to generate a surgical video signal calculating a plurality of AF evaluation values on the basis of the surgical video signal output from the medical imaging device, calculating focus lens moving positions corresponding to respective AF evaluation values on the basis of the plurality of calculated AF evaluation values, and setting at least two of the calculated focus lens moving positions for the assignable button.

A program according to an aspect of the present disclosure is a program for causing a computer to function as a control device configured to calculate a plurality of AF evaluation values on the basis of the surgical video signal output from a medical imaging device including an assignable button to which execution of a selected function is assignable and configured to image an operative field during surgery to generate a surgical video signal, calculate focus lens moving positions corresponding to respective AF evaluation values on the basis of the plurality of calculated AF evaluation values, and set at least two of the calculated focus lens moving positions for the assignable button.

In the medical imaging system, the control method, and the program according to an aspect of the present disclosure, a plurality of AF evaluation values is calculated on the basis of a surgical video signal output from a medical imaging device, focus lens moving positions corresponding to respective AF evaluation values are calculated on the basis of the plurality of calculated AF evaluation values, and at least two of the calculated focus lens moving positions are set for an assignable button included in the medical imaging device.

Note that the medical imaging device and the control device included in the medical imaging system according to an aspect of the present disclosure may be independent devices or may be internal blocks constituting one device.

### BRIEF DESCRIPTION OF DRAWINGS

Fig. 1 is a diagram illustrating a configuration example of a medical imaging system to which a technology according to the present disclosure is applied.
Fig. 2 is a block diagram illustrating a detailed configuration example of a camera head and a control device.
Fig. 3 is a diagram illustrating a configuration example of a control program.
Fig. 4 is a perspective view illustrating a configuration example of a camera head.
Fig. 5 is a flowchart illustrating a first example of an assignable button setting process.
Fig. 6 is a diagram illustrating a calculation example of an AF evaluation value.
Fig. 7 is a flowchart illustrating a second example of an assignable button setting process.
Fig. 8 is a view illustrating a setting example of an AF evaluation frame.
Fig. 9 is a flowchart for describing an assignable button operation handling process.
Fig. 10 is a diagram illustrating another configuration example of the medical imaging system to which the technology according to the present disclosure is applied.
Fig. 11 is a diagram illustrating a configuration example of a microscopic surgery system to which the technology according to the present disclosure is applied.
Fig. 12 is a block diagram illustrating a configuration example of an imaging system to which the technology according to the present disclosure is applied.
Fig. 13 is a block diagram illustrating a hardware configuration of a control device.

### MODE FOR CARRYING OUT THE INVENTION

### <System configuration>

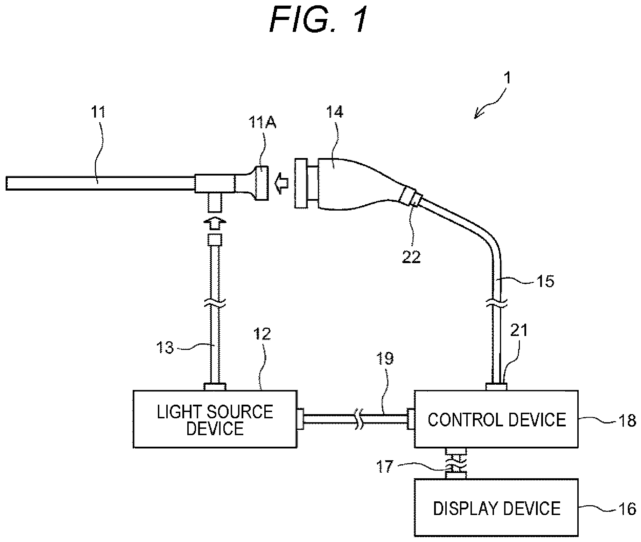

Fig. 1 is a diagram illustrating a configuration example of a medical imaging system to which the technology according to the present disclosure is applied. Fig. 1 illustrates an endoscopic surgery system used in endoscopic surgery of an abdomen as a medical imaging system. Endoscopic surgery is surgery performed in place of conventional laparotomy in a medical field.

A medical imaging system 1 includes an insertion unit 11, a light source device 12, a light guide 13, a camera head 14, a transmission cable 15, a display device 16, a transmission cable 17, a control device 18, and a transmission cable 19. The camera head 14 is an example of a medical imaging device to which the technology according to the present disclosure is applied. The control device 18 is an example of a control device to which the technology according to the present disclosure is applied.

The insertion unit 11 is a member that has a rigid elongated shape as a whole and is inserted into a living body. An optical system that includes one or a plurality of lenses and condenses a subject image is provided in the insertion unit 11. Note that the insertion unit 11 and the camera head 14 may be integrated.

One end of the light guide 13 is detachably connected to the light source device 12, and the other end is detachably connected to the insertion unit 11. The light source device 12 supplies light for illuminating the inside of the living body to one end of the light guide 13 under the control of the control device 18. The light guide 13 transmits the light supplied from the light source device 12 from one end to the other end, and supplies the light to the insertion unit 11.

The light supplied to the insertion unit 11 is emitted from the distal end of the insertion unit 11 and radiated into the living body. The light (subject image) including the light radiated into the living body and reflected in the living body is collected by the optical system in the insertion unit 11. The camera head 14 is detachably connected to the eyepiece unit 11A which is the proximal end of the insertion unit 11. Note that the subject image may include light emission from a living body or a reagent absorbed by the living body, or light from a light source different from the light source device 12.

Under the control of the control device 18, the camera head 14 captures the subject image condensed by the insertion unit 11 to output a surgical video signal (for example, a RAW signal) obtained by the imaging. The surgical video signal is, for example, a video signal corresponding to a video with 4K resolution (3840 pixels × 2160 pixels) or higher. In the following description, (an image frame of) a video based on the surgical video signal is also referred to as a surgical image.

One end of the transmission cable 15 is detachably connected to the control device 18 via a connector 21, and the other end is detachably connected to the camera head 14 via a connector 22. The transmission cable 15 transmits a surgical video signal and the like output from the camera head 14 to the control device 18. In addition, the transmission cable 15 transmits a control signal, a synchronization signal, a clock signal, power, and the like output from the control device 18 to the camera head 14.

Note that the transmission of the surgical video signal and the like from the camera head 14 to the control device 18 via the transmission cable 15 may be either an electric signal or an optical signal. The similar configuration is applied to transmission of a control signal, a synchronization signal, and a clock signal from the control device 18 to the camera head 14 via the transmission cable 15.

One end of the transmission cable 17 is detachably connected to the display device 16, and the other end is detachably connected to the control device 18. The transmission cable 17 transmits the surgical video signal processed by the control device 18, the control signal output from the control device 18, and the like to the display device 16. The display device 16 displays a surgical image based on the surgical video signal output from the camera head 14 under the control of the control device 18.

The control device 18 is configured as, for example, a camera control unit (CCU), and includes a processor such as a central processing unit (CPU), a field programmable gate array (FPGA), a storage device, and the like. The control device 18 outputs a control signal or the like to respective devices including the light source device 12, the camera head 14, and the display device 16, thereby integrally controlling the operation of each device. Furthermore, the control device 18 processes the surgical video signal and the like output from the camera head 14, and controls the operation of each device on the basis of the processing result.

One end of the transmission cable 19 is detachably connected to the light source device 12, and the other end is detachably connected to the control device 18. The transmission cable 19 transmits a control signal and the like from the control device 18 to the light source device 12.

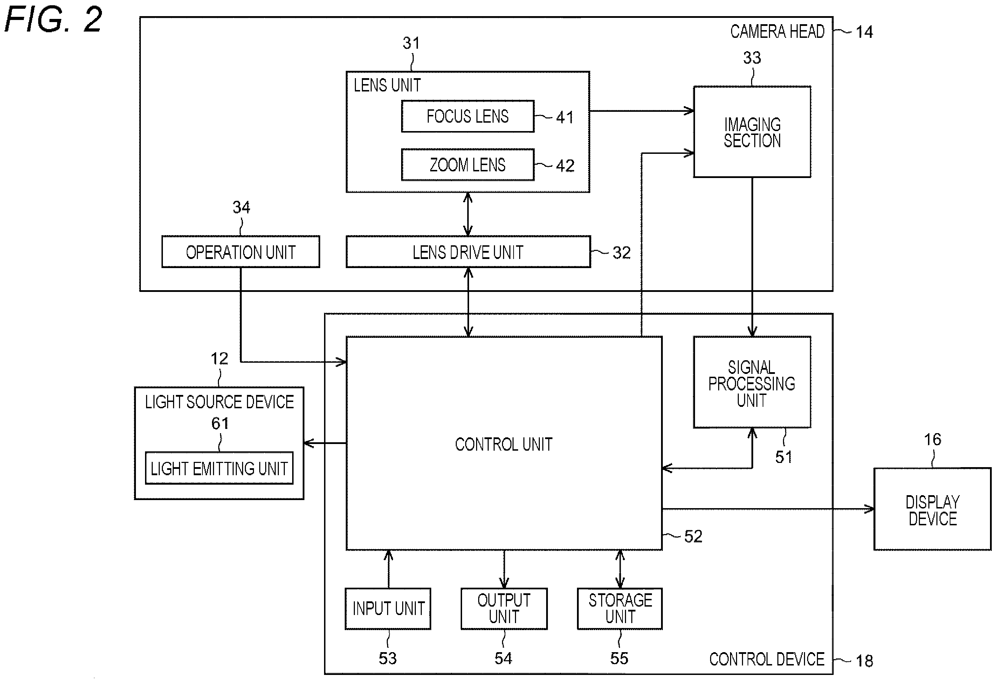

Next, a detailed configuration example of the camera head 14 and the control device 18 will be described with reference to Fig. 2. The camera head 14 includes the lens unit 31, the lens drive unit 32, an imaging section 33, and an operation unit 34.

The lens unit 31 includes a plurality of lenses movable along the optical axis, and forms the subject image condensed by the insertion unit 11 on the imaging face of the imaging section 33. The lens unit 31 includes a focus lens 41 and a zoom lens 42.

The focus lens 41 includes one or a plurality of lenses, and adjusts the focus of the camera head 14 by moving along the optical axis. The zoom lens 42 includes one or a plurality of lenses, and adjusts the angle of view of the camera head 14 by moving along the optical axis.

Although not illustrated, the lens unit 31 includes a focus mechanism that moves the focus lens 41 along the optical axis and an optical zoom mechanism that moves the zoom lens 42 along the optical axis.

The lens drive unit 32 includes an actuator that operates the focus mechanism and the optical zoom mechanism described above, and a drive unit that drives the actuator. The lens drive unit 32 adjusts the focus and the angle of view of the lens unit 31 under the control of the control device 18. In addition, the lens drive unit 32 includes a position sensor such as a photo interrupter. The lens drive unit 32 detects a position of the focus lens 41 and a position of the zoom lens 42 to output a detection signal corresponding to these positions to the control device 18.

The imaging section 33 includes a sensor chip in which an imaging element, a signal processing circuit, and the like are integrally formed. The imaging element is an image sensor such as a charge coupled device (CCD) or a complementary metal oxide semiconductor (CMOS), receives a subject image collected by the insertion unit 11 and formed by the lens unit 31, and converts the subject image into an electric signal. The signal processing circuit performs signal process such as AD conversion on an electric signal (analog signal) from the imaging element. The imaging section 33 images the inside of the living body to output a surgical video signal (digital data) subjected to signal process such as AD conversion according to control from the control device 18.

Note that the signal processing circuit included in the imaging section 33 may be formed separately from the imaging element without being integrally formed with the imaging element. Furthermore, the imaging element preferably has the number of pixels capable of outputting a video signal corresponding to a video with 4K resolution or higher.

The operation unit 34 is configured as a button or the like for performing an operation related to various functions such as an auto focus (AF) function and a manual focus (MF) function. The operation unit 34 outputs an operation signal corresponding to a user's operation to the control device 18. The user includes surgical staff member (operator or the like) who performs surgery on a patient.

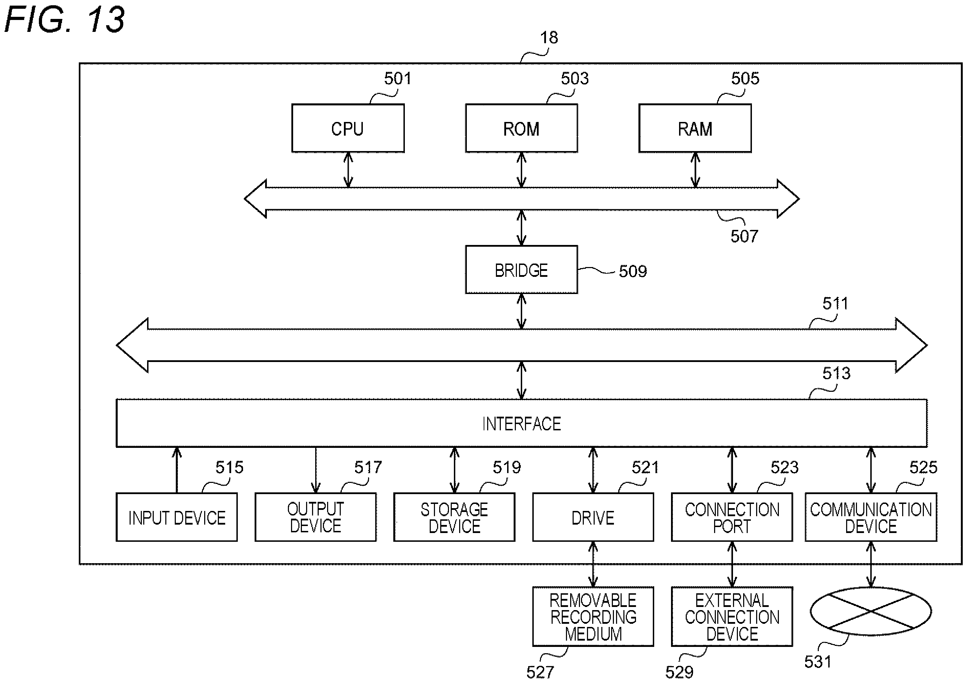

The control device 18 includes a signal processing unit 51, a control unit 52, an input unit 53, an output unit 54, and a storage unit 55. Note that a hardware configuration of the control device 18 will be described later with reference to Fig. 13.

The signal processing unit 51 can be configured by an FPGA and a processor such as a digital signal processor (DSP). The signal processing unit 51 performs RAW processing such as optical black calculation processing and a demosaic process on the surgical video signal from the imaging section 33, and converts the surgical video signal into an RGB signal. The signal processing unit 51 performs an RGB process such as a white balance, an RGB gamma correction, and a YC conversion on the obtained RGB signal. Furthermore, the signal processing unit 51 performs a YC process such as a color difference correction and a noise reduction on the obtained Y and Cb/Cr signals. The signal processing unit 51 supplies the surgical video signal after the signal process to the control unit 52.

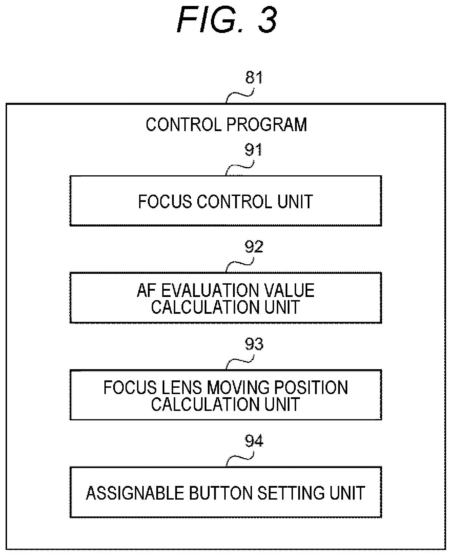

The control unit 52 includes a processor such as a CPU. The control unit 52 controls the operations of respective devices including the light source device 12, the camera head 14, and the display device 16 and controls the operation of the entire control device 18 by outputting a control signal. The control unit 52 can realize various functions (AF function and the like) by reading and executing the control program stored in the storage unit 55. A configuration example of the control program will be described later with reference to Fig. 3.

The control unit 52 controls light emission of a light emitting unit 61 included in the light source device 12. The light emitting unit 61 emits light in order to irradiate a surgical region as a subject with, for example, normal light such as white light imaging (WLI) light (for example, visible light including light having a wavelength band of 360 nm to 760 nm) as light in a first wavelength band (first wavelength light) and special light such as infrared (IR) light as light in a second wavelength band (second wavelength light). The IR light herein may include near infrared (NIR) light. For example, the IR light may be light having a wavelength band of 760 nm or more and 1000 um or less.

The control unit 52 generates a surgical video signal for display on the basis of the surgical video signal processed by the signal processing unit 51 to output the surgical video signal to the display device 16. The display device 16 includes a display including liquid crystal, organic electro luminescence (EL), or the like, and displays a surgical image based on a surgical video signal output from the control device 18.

The input unit 53 includes an operation device such as a button, a switch, a mouse, a keyboard, or a touch panel, and receives a user's operation. The input unit 53 supplies an operation signal corresponding to a user's operation to the control unit 52. The output unit 54 includes a speaker, a printer, and the like to output various types of information.

The storage unit 55 is a storage device such as a semiconductor storage device or a hard disk drive (HDD). The storage unit 55 stores programs such as a control program executed by the control unit 52, information (data, parameters, and the like) necessary for the process of the control unit 52, and the like.

### <Control program configuration>

Fig. 3 is a diagram illustrating a configuration example of a control program 81. The control program 81 is stored in the storage unit 55, and is read and executed by the control unit 52 including a processor such as a CPU. The control program 81 may be executed by the signal processing unit 51.

The control program 81 includes a focus control unit 91, an AF evaluation value calculation unit 92, a focus lens moving position calculation unit 93, and an assignable button setting unit 94.

The focus control unit 91 operates the lens drive unit 32 to adjust the focus of the lens unit 31 (change the position of the focus lens 41). For example, the focus control unit 91 controls the AF operation of the focus lens 41 on the basis of the position of the focus lens 41 detected by the lens drive unit 32 and the AF evaluation value from the AF evaluation value calculation unit 92. Furthermore, the focus control unit 91 controls the AF operation of the focus lens 41 on the basis of an operation signal from the operation unit 34 or the input unit 53.

The AF evaluation value calculation unit 92 calculates a plurality of AF evaluation values on the basis of the surgical video signal from the camera head 14. The AF evaluation value is a focus evaluation value for evaluating a focus state of (subject image in) a surgical image obtained from the surgical video signal.

For example, the AF evaluation value calculation unit 92 detects the contrast and frequency components of the image on the basis of pixel information (for example, luminance data (Y) and Cb/Cr data) in the surgical image of one frame captured by the imaging section 33. The AF evaluation value calculation unit 92 calculates the AF evaluation value on the basis of the detected contrast and frequency components. Alternatively, the AF evaluation value calculation unit 92 may detect the contrast and frequency components of the image in the designated region on the basis of pixel information for each pixel of the designated region (for example, the AF evaluation frame) in the surgical image of one frame.

The focus lens moving position calculation unit 93 calculates moving positions (focus lens moving positions) of the focus lens 41 corresponding to respective AF evaluation values on the basis of a plurality of AF evaluation values. It can also be said that the focus lens moving position calculated in this manner is a focus lens position corresponding to the in-focus position.

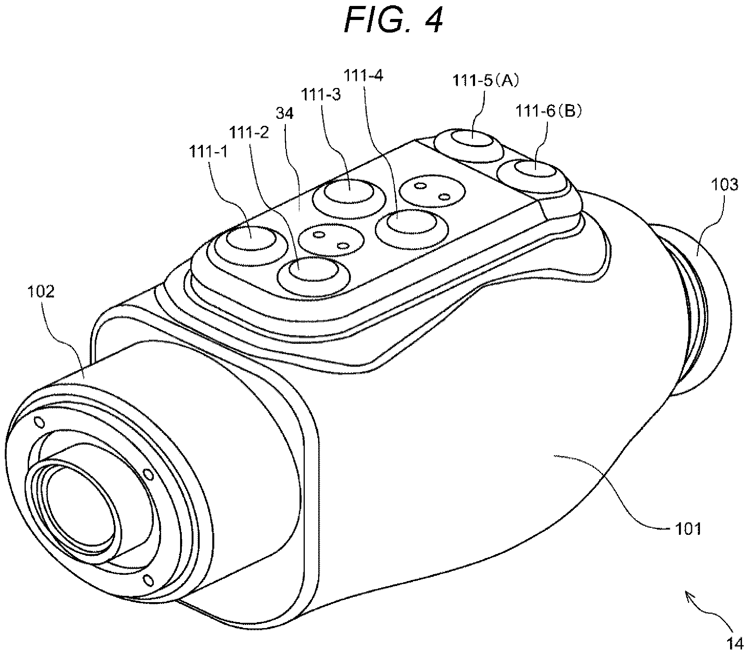

The assignable button setting unit 94 sets at least two of the focus lens moving positions calculated on the basis of a plurality of AF evaluation values for the assignable button. Although details will be described later with reference to Fig. 4 and the like, the assignable button is a button to which execution of the selected function is assignable.

### <Camera head configuration>

Fig. 4 is a perspective view illustrating a configuration example of the camera head 14.

The camera head 14 includes a main body unit 101 having a tubular shape, and a cover unit 102 which is fixed by closing an opening formed in the main body unit 101 and a connector unit 103. The operation unit 34 that outputs an operation signal corresponding to an operation by a user (surgical staff member) to the main body unit 101 is fixed to an upper face of the main body unit 101.

The main body unit 101 has a shape formed in a tubular shape including a material such as metal (titanium, stainless steel, or the like) or alloy in consideration of ease of gripping by a human hand, such as a shape having a curved surface that conforms to the shape of a hand when the user grips the camera head 14. Although not illustrated, the lens unit 31, the lens drive unit 32, and the imaging section 33 are provided inside the main body unit 101.

The cover unit 102 is fixed to the main body unit 101 by closing an opening to which the insertion unit 11 is connected among the openings formed in the main body unit 101. The connector unit 103 is fixed to the main body unit 101 by closing an opening to which the transmission cable 15 is connected among the openings formed in the main body unit 101.

The operation unit 34 includes buttons 111-1 to 111-6 that receive an input of an operation signal by pressing from the outside. The six buttons of the buttons 111-1 to 111-6 output different operation signals. Examples of the operation signal include a signal instructing AF to a predetermined position in the screen, a signal instructing movement of the optical system to the near (short distance) side and the far (long distance) side in MF, and a signal set by the user in any value.

Any of the buttons 111-1 to 111-6 can be used as an assignable button to which execution of the selected function is assignable. In the following description, a case where the button 111-5 is an assignable button A and the button 111-6 is an assignable button B will be described. Note that not only the combination of the buttons 111-5, 111-6 but also, for example, a combination of the buttons 111-1, 111-2 may be the assignable buttons A and B.

### <First example of assignable button setting process>

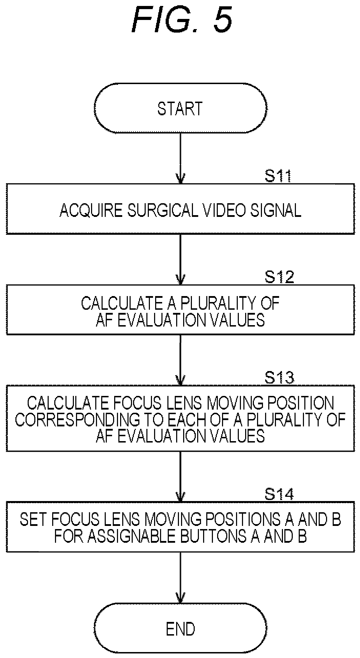

Next, a flow of an assignable button setting process of setting a command to move to the focus lens moving positions A and B for the assignable buttons A and B in Fig. 4 will be described with reference to the flowchart in Fig. 5.

In step S11, the control device 18 acquires the surgical video signal output from the camera head 14. In the control device 18, the control unit 52 or the like executes the control program 81 to perform a process on the surgical video signal from the camera head 14, thereby executing the process of the following steps S12 to S14.

In step S12, the AF evaluation value calculation unit 92 calculates a plurality of AF evaluation values on the basis of the surgical video signal. Examples of a method of calculating the AF evaluation value include contrast AF. The contrast AF is a method of calculating an AF evaluation value on the basis of contrast of a surgical image obtained from a surgical video signal and adjusting the position of the focus lens 41 on the basis of the AF evaluation value. It can also be said that the contrast is a parameter obtained from the surgical image.

Note that the AF evaluation value calculation unit 92 may use another method of calculating the AF evaluation value instead of the contrast AF. For example, the AF evaluation value calculation unit 92 may calculate the AF evaluation value by using a method such as scan AF for calculating a plurality of AF evaluation values by moving the focus lens from a position closes to the near side (Near Max) to a position closes to the far side (Far Max), phase difference AF using a phase difference based on a surgical video signal, image plane phase difference AF using a signal of an image plane phase difference pixel included in the imaging element, distance measurement AF using a distance measurement sensor as a time of flight (ToF) sensor or the like, or spectrum AF for obtaining the AF evaluation value for each wavelength by spectroscopy.

Furthermore, the AF evaluation value calculation unit 92 may combine a plurality of methods of calculating AF evaluation values. Furthermore, the configuration of the camera head 14 may be changed in accordance with the method of calculating the AF evaluation value. For example, in the case of using the phase difference AF, the camera head 14 preferably includes a phase difference optical system. For example, in the case of using the image plane phase difference AF, the camera head 14 preferably includes an imaging element including an image plane phase difference pixel. For example, in a case of using the distance measurement AF, the camera head 14 preferably includes a distance measurement sensor. For example, in the case of using the spectrum AF, the camera head 14 preferably includes a spectroscopic optical system.

Note that the camera head 14 may output information necessary for calculating the AF evaluation value as the surgical video signal according to the method of calculating the AF evaluation value to be used. For example, in the case of using the image plane phase difference AF, the camera head 14 can output a signal including a RAW signal and a phase difference signal as a surgical video signal. For example, in a case of using the distance measurement AF, the camera head 14 can output a signal including a RAW signal and a distance measurement sensor signal as a surgical video signal. The AF evaluation value calculation unit 92 calculates a plurality of AF evaluation values on the basis of the surgical video signal output from the camera head 14 according to the method of calculating the AF evaluation value to be used.

In the contrast AF method, an AF evaluation value based on the contrast of the surgical image is calculated, and the position of the focus lens 41 is adjusted such that the AF evaluation value is the highest or satisfies a predetermined condition. The contrast AF method may include a wobbling operation and a hill-climbing operation. The wobbling operation is an operation of slightly amplifying the focus lens 41 and estimating the direction of the peak of the contrast (the direction in which the focus lens 41 is moved on the optical axis) from the difference between the AF evaluation values at the time of the amplitude. The hill-climbing operation is an operation of searching for a peak position of contrast on the basis of a change in contrast when the focus lens 41 is moved. Here, a plurality of AF evaluation values can be calculated by combining the wobbling operation and the hill-climbing operation.

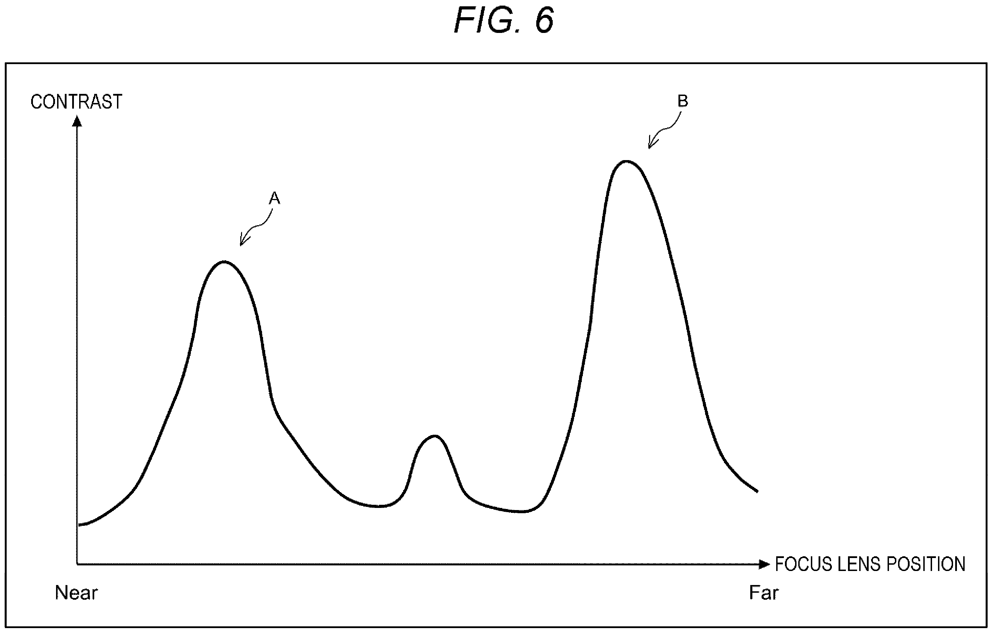

Fig. 6 is a diagram illustrating a calculation example of the AF evaluation value. In Fig. 6, the horizontal axis represents the focus lens position (the left side in the figure is the near side, and the right side is the far side), and the vertical axis represents the contrast. In the contrast AF, the search for the in-focus position is started from the start position after the wobbling operation, and the AF evaluation value based on the contrast is calculated while moving the focus lens 41 in the direction in which the contrast increases. At this time, a peak of the contrast (contrast maximum value) is an in-focus position, and, in the example of Fig. 6, there are two peaks (contrast maximum values) of the peak A and the peak B, and thus, two AF evaluation values based on the peak A and the peak B are calculated.

In step S13, the focus lens moving position calculation unit 93 calculates a focus lens moving position corresponding to each of the plurality of calculated AF evaluation values. In the example of Fig. 6, two focus lens moving positions A and B corresponding to two AF evaluation values based on the peak A and the peak B are calculated, respectively.

In step S14, the assignable button setting unit 94 sets the calculated focus lens moving positions A and B for the assignable buttons A and B. The setting information about the setting of the assignable button can be stored in the storage unit 55.

In the example of Fig. 6, a command to move to the focus lens moving position A corresponding to the AF evaluation value based on the peak A (peak on the near side) is set in the assignable button A (button 111-5 in Fig. 4), and a command to move to the focus lens moving position B corresponding to the AF evaluation value based on the peak B (peak on the far side) is set in the assignable button B (button 111-6 in Fig. 4).

In the example of Fig. 4, the configuration in which the two buttons 111-5, 111-6 are used as the assignable buttons A and B is illustrated, but one or a plurality of assignable buttons can be provided in the operation unit 34 of the camera head 14. In a case where the camera head 14 has one assignable button, the assignable button setting unit 94 sets at least two or more focus lens moving positions for the assignable button. In addition, in a case where the camera head 14 has a plurality of assignable buttons, the assignable button setting unit 94 sets at least one focus lens moving position for each of the assignable buttons.

For example, by using a toggle button or the like as an assignable button, a command to move to two focus lens moving positions A and B can be set for one assignable button A. In a case where such a setting is made, control is only required to be performed so that in a case where the assignable button A is pressed first, the focus lens 41 moves to the focus lens moving position A, and in a case where the assignable button A is pressed next, the focus lens 41 moves to the focus lens moving position B. With respect to the subsequent operation of the assignable button A, similarly, the movement to the focus lens moving position A and the movement to the focus lens moving position B can be alternately repeated.

While, in the example of Fig. 6, two AF evaluation values are calculated, the number of calculated AF evaluation values may be two or more. For example, in a case where three AF evaluation values based on the peak A, the peak B, and the peak C are calculated, a command to move to the focus lens moving positions A, B, and C corresponding to the three calculated AF evaluation values can be set to the assignable button. At this time, three assignable buttons corresponding to the three focus lens moving positions A, B, and C may be prepared, or a command to move to the three focus lens moving positions A, B, and C may be set for two or less assignable buttons.

For example, when the focus lens moving positions A, B, and C corresponding to each of the three AF evaluation values are calculated from the near side to the far side, a command to move to the focus lens moving positions A, B, and C can be set for one assignable button A. In a case where such a setting is made, the focus lens 41 moves from the near side to the far side by moving to the focus lens moving position A in a case where the assignable button A is pressed once, moving to the focus lens moving position B in a case where the assignable button A is pressed twice, and moving to the focus lens moving position C in a case where the assignable button A is pressed three times. Then, when the assignable button A is pressed four times, the focus lens 41 returns to the focus lens moving position A. That is, the focus lens position is sequentially switched according to the number of times the assignable button is pressed.

Alternatively, when using a pair of buttons such as an up button and a down button as the two assignable buttons A and B, in a case where the focus lens moving positions A, B, and C are calculated from the near side to the far side, the focus lens 41 may move to the near side in a case where the up button is pressed, and the focus lens 41 may move to the far side in a case where the down button is pressed.

Specifically, in a case where the focus lens 41 is at the focus lens moving position C, in a case where the up button is pressed twice, the focus lens 41 moves from the focus lens moving position C to the near side in the order of the focus lens moving positions B and A. Furthermore, in a case where the down button is pressed twice in a case where the focus lens 41 is at the focus lens moving position A, the focus lens 41 moves from the focus lens moving position A to the far side in the order of the focus lens moving positions B and C.

A near priority mode in which a near side focus lens moving position is prioritized and a far priority mode in which a far side focus lens moving position is prioritized is prioritized may be selectable from among the focus lens moving positions calculated corresponding to the plurality of AF evaluation values.

For example, in a case where the near priority mode is selected, it is essential to set, for the assignable button, a command to move to the focus lens moving position closest to the near side (Near Max) among the calculated focus lens moving positions, and the focus lens moving position closest to the near side can be set as the default focus lens position. In addition, in a case where the far priority mode is selected, it is essential to set, for the assignable button, a command to move to the focus lens moving position closest to the far side (Far Max) among the calculated focus lens moving positions, and the focus lens moving position closest to the far side can be set as the default focus lens position.

Note that, in a case where the focus lens moving position corresponding to each of three or more AF evaluation values is calculated, the focus lens moving position to be automatically set may be selected according to a predetermined rule, such as preferentially setting the focus lens moving position closest to the near side and the focus lens moving position closest to the far side for the assignable button among the calculated focus lens moving positions.

Alternatively, the user may manually select the focus lens moving position (AF evaluation value) to be set for the assignable button from among the calculated focus lens moving positions (AF evaluation values). In manual selection by the user, in a case where there are two AF evaluation values, in a case where a focus lens moving position corresponding to one AF evaluation value is set to the assignable button, a focus lens moving position corresponding to the other AF evaluation value may be automatically set to the assignable button.

As the assignable button, other operation means such as a rotation ring may be used, for example, in addition to a combination of the buttons 111-5, and 111-6 in Fig. 4, a toggle button, and a combination of an up button and a down button. The rotation ring has a rotation operation member rotatably held with respect to a rotation axis, and can switch a focus lens moving position by rotating the rotation operation member at a predetermined rotation angle. In short, the operation means is only required to be any operation means capable of assigning a command to move to two or more focus lens moving positions, and any operation means may be used.

By storing a preset in the storage unit 55 for each mode of the surgical method (surgical method), when the user selects the surgical mode, the focus lens moving position may be automatically set for the assignable button on the basis of the stored preset. Note that, also in a case where the surgical mode is switched by user setting, the focus lens moving position can be automatically set for the assignable button in a similar manner.

By inputting a surgical image obtained from a surgical video signal to a trained model trained by machine training, in a case where a tumor is found, a position of the tumor is set as an attention region, and a command to move to a focus lens moving position focusing on the attention region can be set for the assignable button. The default focus lens position may be set as a focus lens moving position for focusing on the attention region. The trained model can use a deep neural network (DNN) trained with an image as training data as an input and information regarding a tumor as an output.

At this time, by setting the focus lens moving position calculated corresponding to the AF evaluation value to the assignable button, the AF operation to the attention region and the normal AF operation can be switched according to the operation of the assignable button. In addition, in a case where a plurality of tumors is found, each of the plurality of tumors may be set as an attention region, and a command to move to a focus lens moving position to focus on the attention region may be set for the assignable button, so that the attention region to be focused is switched according to the number of times of pressing the assignable button, for example.

The flow of the first example of the assignable button setting process is described above. In this assignable button setting process, a plurality of AF evaluation values is calculated on the basis of the surgical video signal, focus lens moving positions corresponding to respective AF evaluation values are calculated on the basis of the plurality of AF evaluation values, and at least two of the focus lens moving positions are set for the assignable button. As a result, since the focus lens 41 can be moved to at least two focus lens moving positions in response to the operation of the assignable button, it is possible to cope with a case where there is a plurality of regions desired to be focused.

There may be a plurality of attention points in surgery, and in a case of using the medical imaging system 1, in a case where there is a plurality of attention points, a surgical staff member performs surgery while alternately looking at a plurality of regions to be attention points. For example, in laparoscopic cholecystectomy (surgery in which part or all of a gallbladder is resected using an endoscopic surgery system), a surgical staff member performs surgery while alternately looking at a portion to be resected and a portion not to be resected (such as a gallbladder), and these portions can be attention points. In addition, a method of performing visual marking on a plurality of regions by administering a plurality of biomarkers (labels) into a living body or a cell, and a method of setting an attention region by machine training have been proposed. It is assumed that a plurality of attention points exists in these methods.

As described above, in a case where there is a plurality of attention points, it is assumed that there is a plurality of regions desired to be focused (regions that are most focused by AF), and it is difficult for the current AF function on the assumption that there is one region desired to be focused to cope with the case. For example, in the current AF function, the AF evaluation value is calculated by inputting the value of the pixel signal included in one AF evaluation frame virtually set on the surgical image to the AF evaluation expression, the focus lens moving position corresponding to the calculated AF evaluation value is read, and the focus lens is moved on the basis of the read moving position. As a result, an operation (focusing operation) of focusing on a region that the surgical staff member desires to focus on (want to focus on and view) has been executed.

However, as described above, it is not sufficient to perform the focusing operation only on such one region, and it has been required to provide an AF function that can cope with a case where there is a plurality of regions desired to be focused. Therefore, in the technology according to the present disclosure, a plurality of AF evaluation values is calculated, and at least two of the focus lens moving positions corresponding to respective AF evaluation values are assigned to the assignable button, so that it is possible to cope with a case where there is a plurality of regions desired to be focused. In addition, it is possible to easily switch the focus position only by operating the assignable button according to the determination by the surgical staff member, and the surgical staff member can view the video of the surgery without stress.

### <Second example of assignable button setting process>

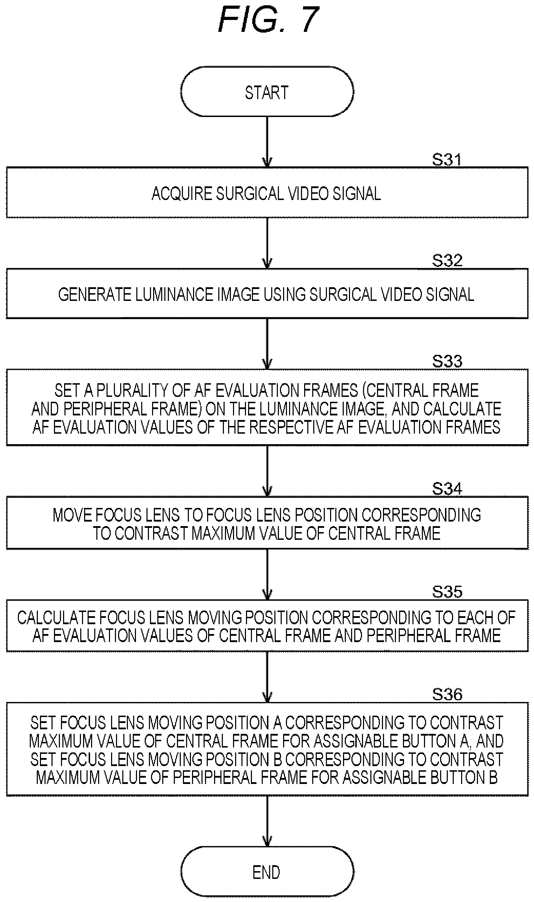

Next, another example of the assignable button setting process will be described with reference to the flowchart of Fig. 7.

In step S31, the control device 18 acquires the surgical video signal (for example, RAW signal) output from the camera head 14. In the control device 18, processing is performed on the surgical video signal from the camera head 14, thereby executing the process of the following steps S32 to S36.

In step S32, the AF evaluation value calculation unit 92 generates a luminance image (Y image) using the surgical video signal from the camera head 14. For example, as a method of generating a luminance image, a luminance image can be generated by performing a demosaic process on a RAW signal acquired as a surgical video signal, or a luminance image can be generated by performing a luminance image generation process after weighting each of an R signal, a G signal, and a B signal included in the RAW signal and adjusting a value.

In step S33, the AF evaluation value calculation unit 92 sets a plurality of virtual AF evaluation frames on the luminance image, and calculates the AF evaluation values of the respective AF evaluation frames. For example, an AF evaluation value of the central frame which is the AF evaluation frame set in the central portion of the luminance image and an AF evaluation value of the peripheral frame which is the AF evaluation frame set in the periphery of the central portion are calculated.

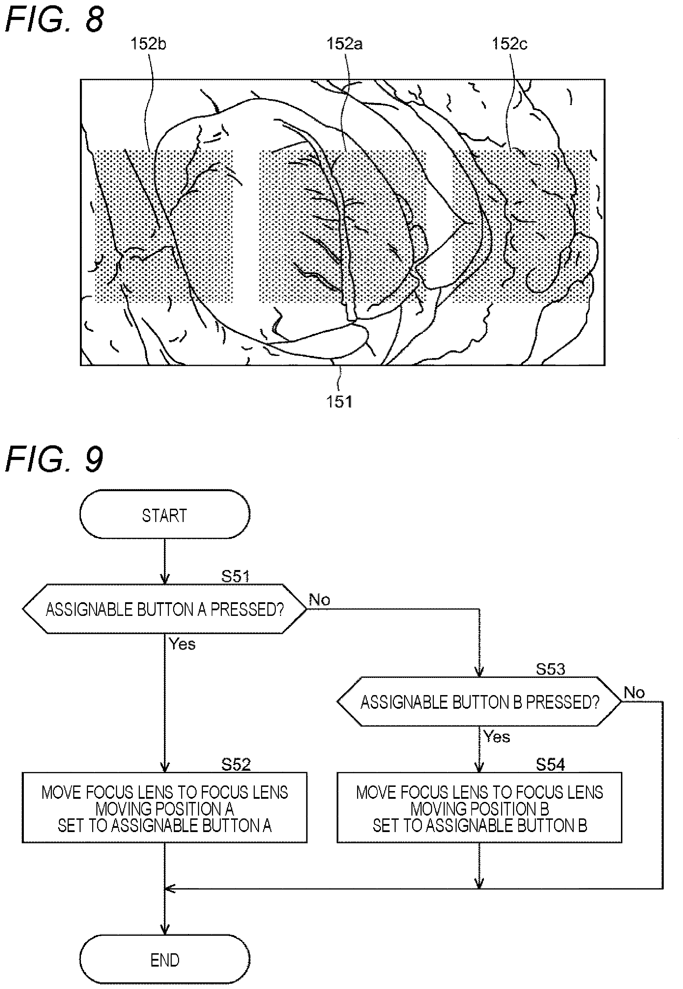

Fig. 8 is a diagram illustrating a setting example of an AF evaluation frame. In Fig. 8, three AF evaluation frames 152a to 152c are set on a luminance image 151. For example, the AF evaluation frame 152a is a central frame set in the central portion of the luminance image 151, the AF evaluation frame 152b and the AF evaluation frame 152c are peripheral frames set on the left and right of the central portion, and the AF evaluation value is calculated for each of the AF evaluation frames 152a to 152c.

As a method of calculating the AF evaluation value, for example, the AF evaluation value can be calculated using various parameters obtained from the surgical image (luminance image or the like), such as an average or variance of the luminance values in the AF evaluation frame, a maximum value or a minimum value of the luminance values in the vertical direction, and a maximum value or a minimum value of the luminance values in the horizontal direction.

In step S34, the focus control unit 91 operates the lens drive unit 32 to move the focus lens 41 to the focus lens position corresponding to the contrast maximum value of the central frame.

Here, for example, by performing contrast AF including the wobbling operation and the hill-climbing operation in the central frame, the AF evaluation value of the central frame is calculated, and it is possible to focus on the contrast maximum value in the central frame. That is, the default focus lens position is a focus lens position corresponding to the contrast maximum value of the central frame. At this time, the AF evaluation value calculation unit 92 constantly calculates the AF evaluation value for the peripheral frame to calculate the contrast maximum value in the peripheral frame.

In step S35, the focus lens moving position calculation unit 93 calculates a focus lens moving position A corresponding to the contrast maximum value of the central frame and a focus lens moving position B corresponding to the contrast maximum value of the peripheral frame.

In step S36, the assignable button setting unit 94 sets a command to move to the focus lens moving position A corresponding to the contrast maximum value of the central frame for the assignable button A (for example, button 111-5 of Fig. 4), and sets a command to move to the focus lens moving position B corresponding to the contrast maximum value of the peripheral frame for the assignable button B (for example, button 111-6 of Fig. 4). The setting information about the setting of the assignable button can be stored in the storage unit 55.

In the second example, as in the first example described above, the number of assignable buttons is not limited to two, and the number of the focus lens moving positions calculated on the basis of a plurality of AF evaluation values is not limited to two, and the description thereof will be omitted because it is repeated. Furthermore, in the second example, in addition to the buttons 111-5, and 111-6 in Fig. 4, a toggle button, a combination of an up button and a down button, a rotation ring, or the like may be used as the operation means, and any operation means may be used.

The flow of the second example of the assignable button setting process is described above. In this assignable button setting process, a plurality of AF evaluation frames is set on the surgical image, AF evaluation values of the plurality of AF evaluation frames are calculated, a focus lens moving position corresponding to a contrast maximum value is calculated for each of the AF evaluation frames on the basis of the AF evaluation values, and at least two of the focus lens moving positions are set for the assignable button. As a result, since the focus lens 41 can be moved to at least two focus lens moving positions in response to the operation of the assignable button, it is possible to cope with a case where there is a plurality of regions desired to be focused.

### <Assignable button operation handling process>

Next, a flow of an assignable button operation handling process will be described with reference to a flowchart of Fig. 9. When the assignable button operation handling process is executed, it is assumed that the assignable button setting process of Fig. 5 or Fig. 7 is executed and a command to move to the focus lens moving positions A and B is set for the assignable buttons A and B.

In step S51, the control unit 52 determines whether or not the assignable button A has been pressed on the basis of an operation signal from the operation unit 34 of the camera head 14.

In a case where it is determined in step S51 that the assignable button A has been pressed, the process proceeds to step S52. In step S52, the focus control unit 91 operates the lens drive unit 32 on the basis of the setting information stored in the storage unit 55 to move the focus lens 41 to the focus lens moving position A set in the assignable button A.

On the other hand, in a case where it is determined in step S51 that the assignable button A has not been pressed, the process proceeds to step S53. In step S53, the control unit 52 determines whether or not the assignable button B has been pressed on the basis of an operation signal from the operation unit 34 of the camera head 14.

In a case where it is determined in step S53 that the assignable button B has been pressed, the process proceeds to step S54. In step S54, the focus control unit 91 operates the lens drive unit 32 on the basis of the setting information stored in the storage unit 55 to move the focus lens 41 to the focus lens moving position B set on the assignable button B.

In a case where the process of step S52 or S54 ends, or in a case where it is determined in the determination process of step S53 that the assignable button B has not been pressed, the series of processes ends.

The flow of the assignable button operation handling process is described above. In the assignable button operation handling process, since the focus lens 41 can be moved to at least two focus lens moving positions in response to the operation of the assignable button by the surgical staff member, it is possible to handle a case where there is a plurality of regions desired to be focused. For example, the surgical staff member can operate the assignable button to perform surgery while alternately looking at a plurality of regions (for example, a portion to be resected and a portion not to be resected).

### <CONFIGURATION including flexible endoscope>

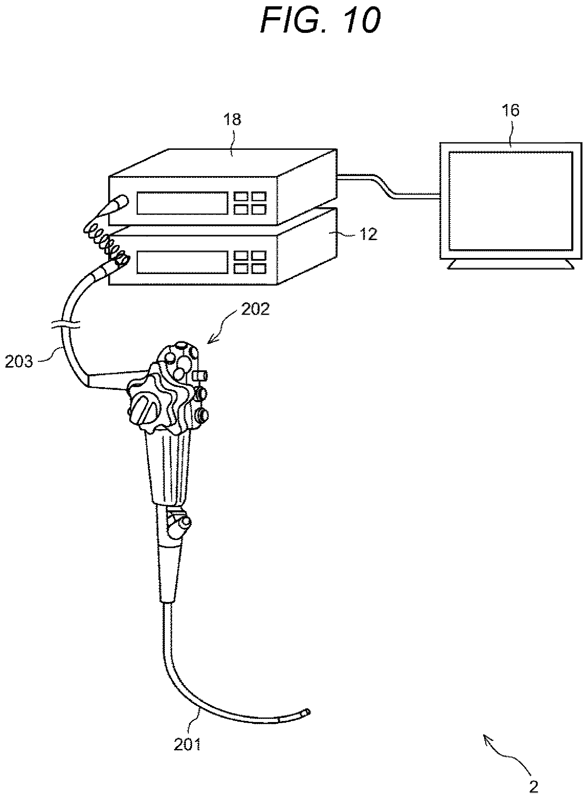

In Fig. 1, the endoscopic surgery system including the rigid endoscope having the insertion unit 11 in which a portion to be inserted into a living body is rigid (not bent) is illustrated as the medical imaging system 1, but a flexible endoscope may be used instead of the rigid endoscope. Fig. 10 illustrates an endoscopic surgery system including a flexible endoscope as a medical imaging system.

A medical imaging system 2 includes an insertion unit 201 in which a portion to be inserted into a living body is flexible (bent), and a camera head 202 that captures a subject image light collected by the insertion unit 201 to output a surgical video signal. As the insertion unit 201, a fiberscope or the like that guides light (subject image) captured by the optical system at the distal end to an eyepiece unit outside the body with a glass fiber and observes the light can be used. The camera head 202 is provided with assignable buttons A and B as in the camera head 14 illustrated in Fig. 4.

The medical imaging system 2 further includes a light source device 12, a display device 16, and a control device 18. The light source device 12 supplies light for illuminating the inside of the living body to a connection unit 203 under the control of the control device 18. The control device 18 receives and processes the surgical video signal output from the camera head 202 via the connection unit 203, and comprehensively controls the operations of the light source device 12, the display device 16, and the camera head 202 on the basis of the processing result.

In the medical imaging system 2, the control device 18 can execute the assignable button setting process of Fig. 5 or 7 on the basis of the surgical video signal from the camera head 202. Furthermore, the control device 18 can execute the assignable button operation handling process of Fig. 9 on the basis of the operation signal from the camera head 202.

In the medical imaging system 2 configured as described above, in a case of performing an endoscopic surgery using an endoscopic surgery system including a flexible endoscope, in a case where there is a plurality of regions desired to be focused, the focus lens moving positions corresponding to respective AF evaluation values of the regions can be set to the assignable button. Therefore, the surgical staff member can focus on any of the plurality of regions desired to be focused by operating the assignable button, and can perform surgery while alternately looking at the plurality of regions.

### <Configuration of microscopic surgery system>

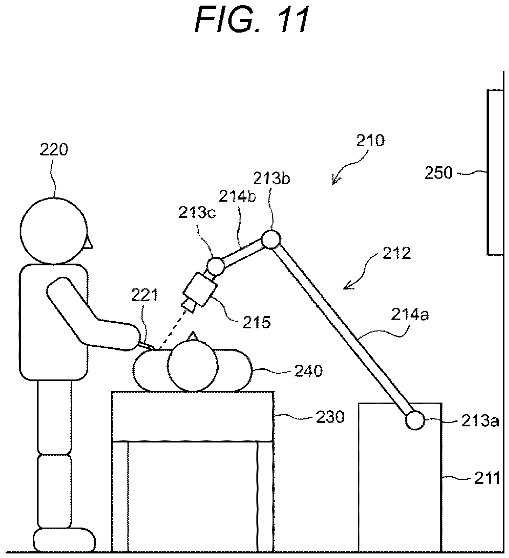

Next, another application example of the medical imaging system to which the technology according to the present disclosure is applied will be described. Fig. 11 illustrates a microscopic surgery system including a surgical video microscope device 210 as an observation medical device that observes the inside of a patient's body.

Fig. 11 illustrates a state in which a doctor 220 is performing surgery on a patient 240 on an operation table 230 using a surgical instrument 221 such as a scalpel, a tweezer, or forceps. In the example of Fig. 11, a state of surgery is illustrated as an example of an operation, but the operation using the surgical video microscope device 210 is not limited to surgery, and may be other various operations (medical treatments such as examinations).

The surgical video microscope device 210 is provided beside the operation table 230. The surgical video microscope device 210 includes a base unit 211 which is a base, an arm unit 212 extending from the base unit 211, and an imaging unit 215 connected to a distal end of the arm unit 212 as a distal end unit. The arm unit 212 includes joint units 213a, 213b, and 213c and links 214a and 214b connected by the joint units 213a to 213c, and the driving of the arm unit 212 is controlled by driving the rotation of the joint units 213a to 213c.

The imaging unit 215 is a unit that acquires an image to be captured by including an optical system that acquires an optical image of a subject, and is configured as, for example, a camera or the like capable of capturing a moving image or a still image. In the example of Fig. 11, postures and positions of the arm unit 212 and the imaging unit 215 are controlled by the surgical video microscope device 210 such that the imaging unit 215 provided at the distal end of the arm unit 212 images the state of the operation site of the patient 240. Note that the configuration of the imaging unit 215 connected to the distal end of the arm unit 212 as a distal end unit is not particularly limited, and may be configured as, for example, an endoscope or a microscope.

A display device 250 having a display is installed at a position facing the doctor 220. The surgical image acquired by the imaging unit 215 is subjected to various types of signal process by a signal processing device built in or externally attached to the surgical video microscope device 210, for example, and then displayed on the display device 250. Consequently, the doctor 220 can perform the surgery while looking at the surgical image displayed on display device 250.

In the microscopic surgery system configured as described above, the imaging unit 215 includes, for example, the camera head 14 and the light source device 12 (light emitting unit 61) in Fig. 2. As in the camera head 14 illustrated in Fig. 4, assignable buttons A and B can be provided on the housing face of the imaging unit 215. The assignable buttons A and B may be provided on a face other than the housing face of the imaging unit 215. The display device 250 corresponds to the display device 16 in Fig. 2.

Furthermore, the signal processing device that performs various types of signal processes on the surgical image acquired by the imaging unit 215 corresponds to the control device 18 in Fig. 2. The signal processing device can execute the assignable button setting process of Fig. 5 or 7 on the basis of the surgical video signal from the imaging unit 215. Furthermore, the control device 18 can execute the assignable button operation handling process of Fig. 9 on the basis of the operation signal from the imaging unit 215.

As described above, in the microscopic surgery system including the surgical video microscope device including the arm, in a case where there is a plurality of regions desired to be focused in performing medical treatment such as surgery, the focus lens moving positions corresponding to respective AF evaluation values of the regions can be set to the assignable button. Therefore, the surgical staff member such as the doctor 220 can focus on any region of the plurality of regions desired to be focused by operating the assignable button, and can perform medical treatment such as surgery while alternately looking at the plurality of regions.

### <Imaging system configuration>

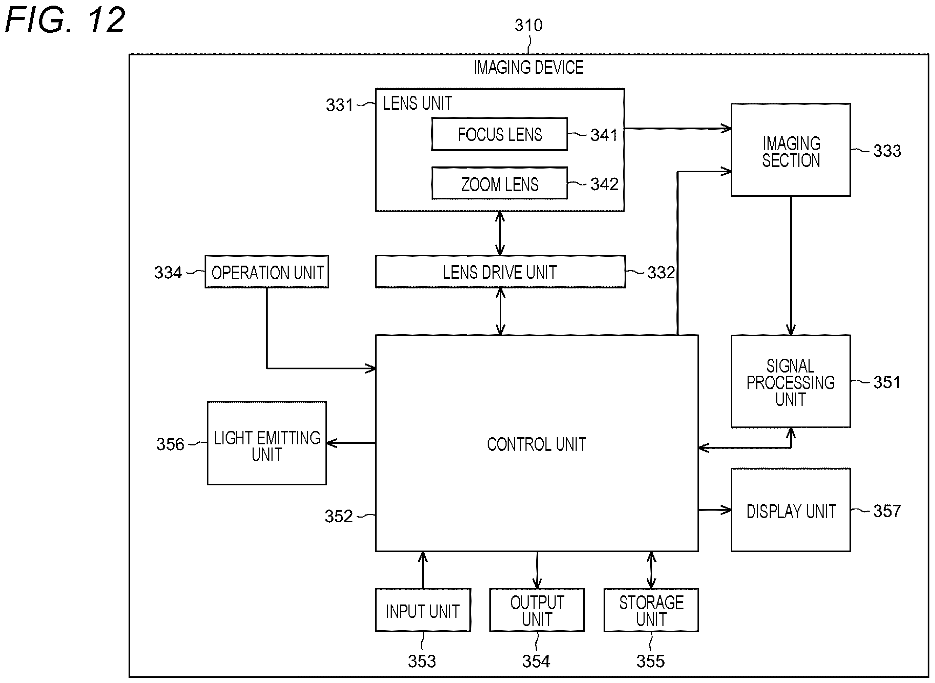

The technology according to the present disclosure can be applied not only to a medical imaging system but also to a general imaging system. Fig. 12 illustrates a configuration example of an imaging device which is an example of a general imaging system. In Fig. 12, an imaging device 310 is configured as, for example, a consumer camera.

The imaging device 310 includes a lens unit 331, a lens drive unit 332, an imaging section 333, an operation unit 334, a signal processing unit 351, a control unit 352, an input unit 353, an output unit 354, a storage unit 355, a light emitting unit 356, and a display unit 357.

The lens unit 331, the lens drive unit 332, the imaging section 333, and the operation unit 334 correspond to the lens unit 31, the lens drive unit 32, the imaging section 33, and the operation unit 34 included in the camera head 14 of Fig. 2, respectively.

A focus lens 341 and a zoom lens 342 included in the lens unit 331 also correspond to the focus lens 41 and the zoom lens 42 included in the lens unit 31 in Fig. 2, respectively. As in the operation unit 34 of the camera head 14 illustrated in Fig. 4, the operation unit 334 is fixed to the housing face of the imaging device 310 and includes assignable buttons A and B.

The signal processing unit 351, the control unit 352, the input unit 353, the output unit 354, and the storage unit 355 correspond to the signal processing unit 51, the control unit 52, the input unit 53, the output unit 54, and the storage unit 55 included in the control device 18 of Fig. 2, respectively. The light emitting unit 356 corresponds to the light emitting unit 61 included in the light source device 12 of Fig. 2. The display unit 357 corresponds to the display device 16 in Fig. 2.

In the imaging device 310, the signal processing unit 351 or the control unit 352 can execute the assignable button setting process of Fig. 5 or 7 on the basis of the video signal from the imaging section 333. In addition, the control unit 352 can execute the assignable button operation handling process of Fig. 9 on the basis of the operation signal from the operation unit 334.

In the imaging device 310 configured as described above, in a case where the imager performs imaging, in a case where there is a plurality of regions desired to be focused, the focus lens moving positions corresponding to respective AF evaluation values of the regions can be set to the assignable button. Therefore, by operating the assignable button, the imager can focus on any region of the plurality of regions desired to be focused, and can perform imaging while alternately looking at the plurality of regions.

### <Hardware configuration>

Fig. 13 is a block diagram illustrating a hardware configuration of the control device 18 constituting the medical imaging system 1 of Fig. 1.

As illustrated in Fig. 13, the control device 18 includes a CPU 501, a read only memory (ROM) 503, and a random access memory (RAM) 505, and is connected via a host bus 507. The control device 18 also includes an input device 515, an output device 517, a storage device 519, a drive 521, a connection port 523, and a communication device 525, and is connected to the interface 513. The host bus 507 is connected to the interface 513 via a bridge 509 and an external bus 511.

The CPU 501 functions as an arithmetic processing device and a control device, and controls an entire or part of operation in the control device 18 in accordance with various programs recorded in the ROM 503, the RAM 505, the storage device 519, or a removable recording medium 527. For example, the CPU 501 can implement the functions of the focus control unit 91, the AF evaluation value calculation unit 92, the focus lens moving position calculation unit 93, and the assignable button setting unit 94 by reading and executing the control program 81 (Fig. 3) stored in the storage device 519.

The input device 515 is an operation unit operated by the user, such as, for example, a mouse, a keyboard, a touch panel, a button, a switch, a lever, and a pedal. Furthermore, the input device 515 may be, for example, a remote controller or an external connection device 529 such as a mobile device corresponding to the operation of the control device 18. By operating the input device 515, the user can input various types of data to the control device 18 and instruct a processing operation.

The output device 517 includes a device that can visually or audibly notify the user of obtained information. Specifically, the output device 517 is configured as a display device having a display including liquid crystal, organic EL, or the like, an audio output device such as a lamp, a speaker, or a headphone, a printer device, and the like. The output device 517 outputs, for example, results obtained by various types of processing performed by the control device 18. Note that the light source device 12 or the display device 16 in Fig. 2 may be realized by the output device 517.

The storage device 519 is a data storage device configured as an example of the storage unit 55 of the control device 18. The storage device 519 is configured by, for example, a magnetic storage unit device such as an HDD, a semiconductor storage device, an optical storage device, a magneto-optical storage device, or the like. The storage device 519 stores programs (for example, the control program 81 in Fig. 3) executed by the CPU 501, various pieces of data, and the like.

The drive 521 is a reader/writer for a recording medium, and is built in or externally attached to the control device 18. The drive 521 reads information recorded in the attached removable recording medium 527 such as an optical disk, a semiconductor memory, a magnetic disk, or a magneto-optical disk to output the information to the RAM 505. Furthermore, the drive 521 can also write information to the attached removable recording medium 527.

The connection port 523 is a port for directly connecting the external connection device 529 to the control device 18. Examples of the connection port 523 include a Universal Serial Bus (USB) port, a high-definition multimedia interface (HDMI) (registered trademark) port, an IEEE 1394 port, and a small computer system interface (SCSI). By connecting the external connection device 529 to the connection port 523, the control device 18 directly acquires various pieces of data from the external connection device 529 or provides various pieces of data to the external connection device 529.

The communication device 525 is, for example, a communication interface including a communication device or the like for connecting to a communication network (network) 531. The communication device 525 is, for example, a communication module or the like compatible with wired or wireless local area network (LAN) communication, cellular communication (for example, fifth generation (5G)), short distance wireless communication such as Bluetooth (registered trademark), wireless communication by wireless USB (WUSB), or the like. Furthermore, the communication device 525 may be a router for optical communication, a modem for various communications, or the like.

The communication device 525 can transmit and receive, for example, signals to and from the Internet and other communication devices in accordance with, for example, a predetermined protocol such as TCP/IP. Furthermore, a communication network 531 connected to the communication device 525 may include a network or the like connected in a wired or wireless manner. The communication network 531 may be, for example, the Internet, a home LAN, a mobile communication network, or a communication network in which infrared communication, radio wave communication, or satellite communication is performed.

Each component of the control device 18 described above may be configured using general-purpose hardware, or may be configured using hardware specialized for the function of each component. That is, it is possible to appropriately change the hardware configuration to be used according to the technical level at the time of implementing the technology according to the present disclosure.

Furthermore, a computer program for realizing each function of the control device 18 constituting the medical imaging system 1 to which the technology according to the present disclosure is applied can be produced and mounted on a personal computer or the like. Furthermore, it is also possible to provide a computer-readable removable recording medium 527 in which such a computer program is stored. The computer program may be distributed from the server via the communication network 531, for example, without using the removable recording medium 527.

### <Modifications>

In the second example of the assignable button setting process described above, the AF evaluation value of each of the plurality of AF evaluation frames is calculated by setting the plurality of AF evaluation frames on the surgical image. However, a marking region visually marked by a biomarker as an AF target region may be extracted from the surgical image, an AF evaluation frame including the marking region may be set, and the AF evaluation value of each AF evaluation frame may be calculated.

As a result, the focus lens moving position is calculated for each AF evaluation frame including the marking region, and at least two of the calculated focus lens moving positions are set for assignable buttons. At this time, by weighting the R signal, the G signal, and the B signal of the surgical video signal (RAW signal), for example, a marking region in the R signal and a marking region in the B signal may correspond to regions of the different reagents.

Further, a plurality of AF evaluation values may be calculated by calculating the AF evaluation value of the surface using a WLI image, which is a surgical image obtained by the light emitting unit 61 included in the light source device 12 radiating the WLI light (first wavelength light), and calculating the AF evaluation value of the specific marking region using an IR image, which is a surgical image obtained by the light emitting unit 61 radiating the IR light (second wavelength light).

That is, in the surgical image, the AF evaluation values (a plurality of AF evaluation values) of the first region irradiated with the first wavelength light (for example, WLI light) and the second region irradiated with the second wavelength light (for example, IR light) are calculated, the focus lens moving position corresponding to each of the first region and the second region is calculated on the basis of the calculated AF evaluation values, and at least two of the calculated focus lens moving positions are set for the assignable button. Note that when each image such as a WLI image or an IR image is used, the evaluation value may be calculated by changing the wavelength of light to be radiated. In addition, as the AF target region, a plurality of attention regions may be extracted using a trained model trained by machine training, and the AF evaluation value may be calculated for each of the extracted attention regions.

In the method of calculating the AF evaluation value described above, the calculation method may be switched according to the surgical mode or the observation mode. The setting position of the AF evaluation frame described above may also be switched according to the surgical mode or the observation mode. That is, the AF parameters such as the method of calculating the AF evaluation value and the setting position of the AF evaluation frame can be switched according to the mode such as the surgical mode or the observation mode. Furthermore, the AF characteristics (AF speed, various threshold values, and the like) may be changed on the basis of any of the surgical mode, the observation mode, and the relationship (area, arrangement, and the like) between the AF target regions.

Furthermore, the priority of the AF target region (default target region or focus lens position to be adjusted first) may be set on the basis of the relationship (area, arrangement, and the like) between the AF target regions. Alternatively, the assignment of the AF target region may be automatically set on the basis of the relationship between the AF target regions. For example, in a case where one organ is dyed in two colors by a biomarker or the like, which of the two colors is set as a default, whether or not to set the focus lens moving position to the assignable button A, and the like are automatically set.

Note that embodiments of the present disclosure are not limited to the embodiment described above, and various modifications may be made without departing from the scope of the present disclosure. The effects described in the present specification are merely examples and are not limited, and other effects may be provided.

Furthermore, each step described in the flowchart described above can be performed by one device or by a plurality of devices in a shared manner. Moreover, in a case where a plurality of processing is included in one step, a plurality of the processing included in the one step can be performed by one device or by a plurality of devices in a shared manner.

In the present description, a system means a set of a plurality of components (devices, modules (parts), and the like), and it does not matter whether or not all the components are in the same housing. Therefore, a plurality of devices housed in separate housings and connected via a network, and a single device with a plurality of modules disposed in one housing are both systems.

Furthermore, the present disclosure can have the following configurations.

(1) A medical imaging system including
   a medical imaging device including an assignable button to which execution of a selected function is assignable and configured to image an operative field during surgery to generate a surgical video signal, and
   a control device configured to control the medical imaging device,
   in which the control device includes one or more processors and one or more storage devices storing a program, and
   the processor executes the program to
   calculate a plurality of AF evaluation values on the basis of the surgical video signal output from the medical imaging device,
   calculate focus lens moving positions corresponding to respective AF evaluation values on the basis of the plurality of calculated AF evaluation values, and
   set at least two of the calculated focus lens moving positions for the assignable button.
(2) The medical imaging system according to Item (1), in which the medical imaging device includes one or a plurality of the assignable buttons.
(3) The medical imaging system according to Item (2),
   in which, in a case where the medical imaging device includes a plurality of the assignable buttons, the processor sets at least one of the focus lens moving positions for each of the assignable buttons.
(4) The medical imaging system according to Item (2),
   in which, in a case where the medical imaging device includes one of the assignable buttons, the processor sets at least two or more of the focus lens moving positions for the assignable button.
(5) The medical imaging system according to any one of Items (1) to (4),
   in which, in a case where the assignable button is operated, the processor moves a focus lens included in the medical imaging device to the focus lens moving position set for the operated assignable button.
(6) The medical imaging system according to any one of Items (1) to (5),
   in which the processor
   calculates the plurality of AF evaluation values on the basis of a parameter of a surgical image obtained from the surgical video signal, and
   calculates the focus lens moving positions corresponding to a plurality of contrast maximum values on the basis of the plurality of calculated AF evaluation values.
(7) The medical imaging system according to Item (6), in which the processor
   sets a plurality of AF evaluation frames on the surgical image,
   calculates an AF evaluation value of each of the plurality of set AF evaluation frames, and
   calculates the focus lens moving position corresponding to a contrast maximum value for each of the AF evaluation frames on the basis of the calculated AF evaluation value.
(8) The medical imaging system according to Item (7),
   in which the plurality of AF evaluation frames includes a central frame set at a central portion on the surgical image and a peripheral frame set around the central portion, and
   the processor
   calculates an AF evaluation value of each of the central frame and the peripheral frame, and
   calculates the focus lens moving position corresponding to a contrast maximum value of the central frame and the focus lens moving position corresponding to a contrast maximum value of the peripheral frame on the basis of the calculated AF evaluation value.
(9) The medical imaging system according to Item (8),
   in which the processor moves the focus lens to a default focus lens position corresponding to a contrast maximum value of the central frame when first moving the focus lens.
(10) The medical imaging system according to Item (9),
   in which the processor controls a wobbling operation of the focus lens when moving the focus lens.
(11) The medical imaging system according to any one of Items (1) to (5),
   in which, in a case where a near side focus lens moving position and a far side focus lens moving position are calculated as the focus lens moving positions corresponding to the plurality of AF evaluation values, the processor sets the near side focus lens moving position and the far side focus lens moving position for the assignable button.
(12) The medical imaging system according to Item (11),
   in which, in a case where the focus lens moving position is calculated from a near side to a far side, the processor sets the focus lens moving position for the assignable button such that the focus lens moves from the near side to the far side.
(13) The medical imaging system according to any one of Items (1) to (5),
   in which the processor
   extracts a plurality of marking regions visually marked by biomarkers as AF target regions from a surgical image obtained from the surgical video signal,
   sets a plurality of AF evaluation frames including the extracted marking regions,
   calculates an AF evaluation value for each of the set AF evaluation frames, and
   calculates the focus lens moving position for each of the AF evaluation frames on the basis of the calculated AF evaluation value.
(14) The medical imaging system according to any one of Items (1) to (5), further including
   a light source device including a light emitting unit capable of irradiating a surgical region with first wavelength light and second wavelength light,
   in which the processor
   calculates an AF evaluation value of each of a first region irradiated with the first wavelength light and a second region irradiated with the second wavelength light, and
   calculates the focus lens moving position corresponding to each of the first region and the second region on the basis of the calculated AF evaluation value.
(15) A control method including
   a control device configured to control a medical imaging device including an assignable button to which execution of a selected function is assignable and configured to image an operative field during surgery to generate a surgical video signal
   calculating a plurality of AF evaluation values on the basis of the surgical video signal output from the medical imaging device,
   calculating focus lens moving positions corresponding to respective AF evaluation values on the basis of the plurality of calculated AF evaluation values, and
   setting at least two of the calculated focus lens moving positions for the assignable button.
(16) A program for causing a computer to function as a control device configured to
   calculate a plurality of AF evaluation values on the basis of the surgical video signal output from a medical imaging device including an assignable button to which execution of a selected function is assignable and configured to image an operative field during surgery to generate a surgical video signal,
   calculate focus lens moving positions corresponding to respective AF evaluation values on the basis of the plurality of calculated AF evaluation values, and
   set at least two of the calculated focus lens moving positions for the assignable button.

### REFERENCE SIGNS LIST

- 1, 2: Medical imaging system
- 11: Insertion unit
- 12: Light source device
- 14: Camera head
- 16: Display device
- 18: Control device
- 31: Lens unit
- 32: Lens drive unit
- 33: Imaging section
- 34: Operation unit
- 41: Focus lens
- 42: Zoom lens
- 52: Control unit
- 81: Control program
- 91: Focus control unit
- 92: AF evaluation value calculation unit
- 93: Focus lens moving position calculation unit
- 94: Assignable button setting unit
- 101: Main body unit
- 111-1 to 111-6: Button
- 201: Insertion unit
- 202: Camera head
- 310: Imaging device
- 331: Lens unit
- 332: Lens drive unit
- 333: Imaging section
- 334: Operation unit
- 351: Signal processing unit
- 352: Control unit
- 341: Focus lens
- 342: Zoom lens
- 501: CPU
- 519: Storage device
- A, B: Assignable button

## Claims

1. A medical imaging system comprising:
a medical imaging device including an assignable button to which execution of a selected function is assignable and configured to image an operative field during surgery to generate a surgical video signal; and
a control device configured to control the medical imaging device,
wherein the control device includes one or more processors and one or more storage devices storing a program, and
the processor executes the program to
calculate a plurality of AF evaluation values on a basis of the surgical video signal output from the medical imaging device,
calculate focus lens moving positions corresponding to respective AF evaluation values on a basis of the plurality of calculated AF evaluation values, and
set at least two of the calculated focus lens moving positions for the assignable button.

2. The medical imaging system according to claim 1,
wherein the medical imaging device includes one or a plurality of the assignable buttons.

3. The medical imaging system according to claim 2,
wherein, in a case where the medical imaging device includes a plurality of the assignable buttons, the processor sets at least one of the focus lens moving positions for each of the assignable buttons.

4. The medical imaging system according to claim 2,
wherein, in a case where the medical imaging device includes one of the assignable buttons, the processor sets at least two or more of the focus lens moving positions for the assignable button.

5. The medical imaging system according to claim 1,
wherein, in a case where the assignable button is operated, the processor moves a focus lens included in the medical imaging device to the focus lens moving position set for the operated assignable button.

6. The medical imaging system according to claim 5,
wherein the processor
calculates the plurality of AF evaluation values on a basis of a parameter of a surgical image obtained from the surgical video signal, and
calculates the focus lens moving positions corresponding to a plurality of contrast maximum values on a basis of the plurality of calculated AF evaluation values.

7. The medical imaging system according to claim 6,
wherein the processor
sets a plurality of AF evaluation frames on the surgical image,
calculates an AF evaluation value of each of the plurality of set AF evaluation frames, and
calculates the focus lens moving position corresponding to a contrast maximum value for each of the AF evaluation frames on a basis of the calculated AF evaluation value.

8. The medical imaging system according to claim 7,
wherein the plurality of AF evaluation frames includes a central frame set at a central portion on the surgical image and a peripheral frame set around the central portion, and
the processor
calculates an AF evaluation value of each of the central frame and the peripheral frame,
calculates the focus lens moving position corresponding to a contrast maximum value of the central frame and the focus lens moving position corresponding to a contrast maximum value of the peripheral frame on a basis of the calculated AF evaluation value.

9. The medical imaging system according to claim 8,
wherein the processor moves the focus lens to a default focus lens position corresponding to a contrast maximum value of the central frame when first moving the focus lens.

10. The medical imaging system according to claim 9,
wherein the processor controls a wobbling operation of the focus lens when moving the focus lens.

11. The medical imaging system according to claim 5,
wherein, in a case where a near side focus lens moving position and a far side focus lens moving position are calculated as the focus lens moving positions corresponding to the plurality of AF evaluation values, the processor sets the near side focus lens moving position and the far side focus lens moving position for the assignable button.

12. The medical imaging system according to claim 11,
wherein, in a case where the focus lens moving position is calculated from a near side to a far side, the processor sets the focus lens moving position for the assignable button such that the focus lens moves from the near side to the far side.

13. The medical imaging system according to claim 5,
wherein the processor
extracts a plurality of marking regions visually marked by biomarkers as AF target regions from a surgical image obtained from the surgical video signal,
sets a plurality of AF evaluation frames including the extracted marking regions,
calculates an AF evaluation value for each of the set AF evaluation frames, and
calculates the focus lens moving position for each of the AF evaluation frames on a basis of the calculated AF evaluation value.

14. The medical imaging system according to claim 5, further comprising:
a light source device including a light emitting unit capable of irradiating a surgical region with first wavelength light and second wavelength light,
wherein the processor
calculates an AF evaluation value of each of a first region irradiated with the first wavelength light and a second region irradiated with the second wavelength light, and
calculates the focus lens moving position corresponding to each of the first region and the second region on a basis of the calculated AF evaluation value.

15. A control method comprising:
a control device configured to control a medical imaging device including an assignable button to which execution of a selected function is assignable and configured to image an operative field during surgery to generate a surgical video signal,
calculating a plurality of AF evaluation values on a basis of the surgical video signal output from the medical imaging device,
calculating focus lens moving positions corresponding to respective AF evaluation values on a basis of the plurality of calculated AF evaluation values, and
setting at least two of the calculated focus lens moving positions for the assignable button.

16. A program for causing a computer to function as a control device configured to
calculate a plurality of AF evaluation values on a basis of the surgical video signal output from a medical imaging device including an assignable button to which execution of a selected function is assignable and configured to image an operative field during surgery to generate a surgical video signal,
calculate focus lens moving positions corresponding to respective AF evaluation values on a basis of the plurality of calculated AF evaluation values, and
set at least two of the calculated focus lens moving positions for the assignable button.
